(19) Europäisches Patentamt
European Patent Office
Office européen des brevets

(11) EP 0 402 477 B1

(12) **EUROPEAN PATENT SPECIFICATION**

(45) Date of publication and mention of the grant of the patent: **19.03.1997 Bulletin 1997/12**

(21) Application number: **90900361.8**

(22) Date of filing: **21.12.1989**

(51) Int. Cl.$^6$: **A61K 31/557**

(86) International application number: **PCT/JP89/01287**

(87) International publication number: **WO 90/07335 (12.07.1990 Gazette 1990/16)**

# (54) USE OF ISOCARBACYCLINS FOR PREVENTING OR TREATING ORGAN DISEASES

VERWENDUNG VON ISOKARBACYCLINEN ZUR VORBEUGUNG ODER BEHANDLUNG VON ORGANKRANKHEITEN

UTILISATION D'ISOCARBACYCLINES POUR LA PREVENTION OU LE TRAITEMENT DE MALADIES D'ORGANES

(84) Designated Contracting States: **AT BE CH DE ES FR GB IT LI NL SE**

(30) Priority: **23.12.1988 JP 323687/88**

(43) Date of publication of application: **19.12.1990 Bulletin 1990/51**

(73) Proprietor: **TEIJIN LIMITED**
**Osaka-shi Osaka 541 (JP)**

(72) Inventors:
- **BANNAI, Kiyoshi**
  **Tokyo 191 (JP)**
- **TANAKA, Toshio**
  **Tokyo 191 (JP)**
- **KATO, Yoshinori**
  **Tokyo 191 (JP)**
- **KOYAMA, Tamotsu**
  **Tokyo 192 (JP)**
- **ASANO, Satoshi**
  **403, Fukiage Green Manshio**
  **Tokyo 191 (JP)**
- **OHTSU, Akira**
  **Tokyo 198 (JP)**
- **KUROZUMI, Seizi**
  **Tokyo 185 (JP)**
- **OGAWA, Makoto**
  **Chiba 280 (JP)**
- **MORI, Yoshio**
  **Chiba 280 (JP)**

(74) Representative: **Kraus, Walter, Dr. et al**
**Patentanwälte Kraus, Weisert & Partner**
**Thomas-Wimmer-Ring 15**
**80539 München (DE)**

(56) References cited:

| | |
|---|---|
| **EP-A- 0 247 740** | **WO-A-86/00808** |
| **JP-A- 6 144 819** | **JP-A-58 203 911** |
| **JP-A-61 197 518** | **JP-A-61 289 034** |

- **PATENT ABSTRACTS OF JAPAN, vol. 11, no. 193 (C-430)[2640], 20th June 1987; & JP-A-62 19 565**
- **PATENT ABSTRACTS OF JAPAN, vol. 11, no. 361 (C-459)[2808], 25th November 1987; & JP-A-62 132 839**
- **PATENT ABSTRACTS OF JAPAN, vol. 13, no. 169 (C-587)[3517], 21st April 1989; & JP-A-64 20**
- **PATENT ABSTRACTS OF JAPAN, vol. 11, no. 24 (C-399)[2471], 23rd January 1987; & JP-A-61 197 518**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 402 477 B1

## Description

The present invention relates to a use of isocarbacyclins in the prevention or therapy of organ disorders, a pharmaceutical composition containing the same and a process for its production.

Prostaglandins (hereinafter sometimes abbreviated as PG) have various physiological actions such as a strong platelet aggregation inhibitory action, an antihypertensive action, an action to inhibit the secretion of gastric acid, a contractive action of smooth muscles, a diuretic action, etc. They are substances useful for the therapy or the prevention of peripheral circulatory disorders, cardiac infarction, angina, arteriosclerosis, hypertension, gastric ulcer, duodenal ulcer, induction of labor, artificial termination of pregnancy and the like.

Recently it has been brought to light that some kind of these prostaglandins have an action of protecting cells of an endogeneous tissue, a so-called cell protecting action. This cell protecting action is present in all endogeneous cells. It is said that for example, a thrapeutic effect on the gastric ulcer, a focal contraction effect of cardiac infarction, a preventive effect of the lung injury involved by an endotoxin shock and the like are attributable to this cell protecting action [see "Igaku no ayumi (a step in medical science), a Japanese material, vol. 125, p.p. 250, 19831. Further, it is known that prostaglandins exhibit a cell protecting action to hepatocells. For instance, it is reported that 16,16-dimethyl-$PGE_2$, a structural modifier of $PGE_2$, is useful to prevent the necrosis of parenchymal liver cells of rats induced by carbon tetrachloride and galactosamine [see "Folia Histochemi et Cytochemica" vol.18, p.p. 311, 1980; and "Gastroenterology", vol.81, p.p. 211, 1981]. Further, regarding an action to protect hepatocytes produced by $PGE_2$ and its structural modifier (15-methyl-$PGE_2$, 16,16-dimethyl-$PGE_2$, etc.) this is mentioned in US Patent Specification No. 4,374,856. Further, we discovered that thia-$PGE_1$s would have an inhibitory action of hepatic disorders and filed an application separately (Japanese Laid-Open Patent Publn. No. 62-129218). Moreover, the following action is reported in Japanese Laid-Open Patent Publns. Nos. 58-164512, 58-203911 and 62-277352, i.e. a cell protecting action exhibited by a structural modifier of 6-oxo-$PGE_1$.

Additionally, it is reported that prostacyclins ($PGI_2$) have an action of protecting a cat's hepatic tissue under an oxgen scarce condition [see "Amer. J. Physiol. vol.238, p.p. 176, 1980]. We discovered that 7-fluoro-$PGI_2$s, a derivative for prostacyclins, would have an inhibitory action of hepatic disorders and filed an application separately (Japanese Laid-Open Patent Publn. No. 63-27433). Furthermore, it is reported that 6,9-alpha-nitrilo-$PGI_1$ also have a hepatic tissue protecting action (see official gazettes of Japanese Laid-Open Patent Publns. No. 58-164512 and 58-203911). And besides, it is reported that iloprost, a carbacyclin derivative, and nileprost, of cyanoprostacyclins, also have an action of protecting a liver, a pancreas and a kidney (see an official gazette of Japanese Announcement No. 61-502819).

Further, in recent years prostaglandins have been utilized in the field of organ transplantation, thanks to its strong cell protectiing action (see "Modern medical science" written by K. Ohta, No. 18, p.p. 2693-2697, 1986). Namely, prostaglandins are used for storing any organ enucleated from a donor to insure the protection of said organ at the time of its transplantation, minimizing any trouble ocurring until the transplantation operation and to ensure the preservation of said organ in a good condition. For instance, it is reported that natural prostacyclins ($PGI_2$) are useful for the preservation of a liver and a kidney (M. Monden et al., Ann. Surg. vol. 196 p.p.38, 1982, J.W. Bradlet et al., Transplan. Proc., vol. 15, p.p. 424, 1983). There are also reports that OP-41483, a derivative for prostacyclin, is useful for the storage of a kidney (M. Tobimatsu et al, Transplant. Proc., vol. 17 p.p. 1461, 1985) and that prostaglandins are useful to inhibit the rejection at the time of organ transplantation by virtue of its strong cell protectiing action ("Modern medical science" written by K. Ohta, No. 18, p.p. 2693-2697, 1986). We discovered that isocarbacyclins of the following formula (I)-a had a protecting action at the time of enucleating such organs from a living human body and filed an application separately (see an official gazette of Japanese Laid-Open Patent Publn. No. 64-20).

By the way, naturally occurring prostacyclins are a local hormone produced in vivo mainly on endothelium of an arteria and an attempt has been made of providing this as a drug directly by utilizing being an important factor which regulates the cell function in vivo depending on its strong physiological activity such as a platelet aggregation inhibitory activity, a vasodilator activity and the like ["Clinical Pharmacology of Prostacyclin" written by P. J. Lewis, J.O. Grady et al, Raven Press, N.Y. 1981]. However, since naturally ocurring prostacyclins have an enol ether bond which is very easy to hydrolyze within the molecule, they are easily deactivated under neutral or acidic conditions and therefore, cannot be said to be a desirable compound because of being chemically unstable as medicines. Thus, ardent studies have been made of chemically stable synthetic prostacyclin derivatives which have the same physiological activity as that of naturally ocurring prostacyclin [see "Synthesis", 1984, p.p. 449]. As a result, the Inventors were successful in the synthesis of 9(O)-methano-$\Delta^{6(9\alpha)}$-prostaglandins $I_1$ (isocarbacyclins), prostacyclins of the following formula (I)-a which fully satisfy chemically stable properties by replacing an oxygen atom in the 6,9 alpha-position of prostacyclin with a methine group (-CH=)(see official gazettes of European Patent Laid-Open Publns. Nos. 216585 and 247740 and US Patent specification No. 4788319).

Among these isocarbacyclins of the formula (I)-a derivatives wherein n=0, in particular, had strong physiological actions such as a platelet aggregation inhibitory action, an antihypertensive action, etc. equal to those of naturally occurring prostacyclins and were useful as a pharmaceutical agent for circulatory organs. Moreover, these isocarbacyclins were useful as an agent for lowering the lipid in blood (see Japanese Laid-Open Patent Publn. No. 63-152319).

While, in the official gazette of Japanese Laid-Open Patent Publn. No. 62-19565 are disclosed 15- or 16-hydroxy-3-thia (iso)carbacyclins and a process for their preparation. It is also disclosed that they have a platelet aggregation inhibitory activity.

Further, regarding 15-hydroxy-3-oxa(iso)carbacyclins are disclosed a process for the identification of these compounds and a process for their preparation in the official gazette of Japanese Laid-Open Patent Publn. No. 62-138448. And concerning 16-hydroxy 3-oxa(iso)carbacyclins are disclosed a process for the identification of these compounds and a process for their prepration in the official gazette of Japanese Laid-Open Patent Publn. No. 62-138448.

Consequently, an object of this invention is to provide a process for using particular isocarbacyclins in the prevention or therapy of organ disorders as well as pharmaceutical compositions containing the same. Another object of the invention is to provide the above process and pharmaceutical compositions on the basis of such discovery that specific isocarbacyclins are excellent in the action to prevent or cure organ disorders in vivo and moreover, they are low in the action on the circulatory system such as an antihypertensive action or a platelet aggregation inhibitory action and consequently, the aimed actional selectivity is high.

A further object of the invention is to provide a process for the prevention or therapy of disorders of organs in vivo, especially a liver or a kidney by using isocarbacyclins which are specially excellent in the action for preventing or curing the above disorders and is to provide pharmaceutical compositions containing the same. A still further object of the invention is to provide the use of isocarbacyclins in the preparation of pharmaceutical compositions which are useful for the above prevention or therapy of organ disorders.

The present invention provides a pharmaceutical composition useful for the prevention or therapy of hepatic diseases, kidney diseases, pancreatic diseases and diseases of stomach, heart or lung, which contains isocarbacyclins of the following formula:

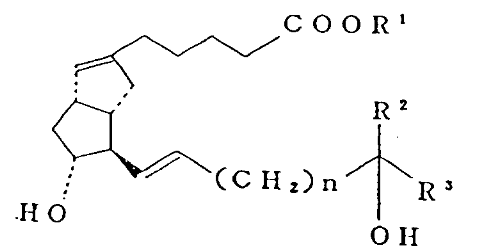

(I-)-a

wherein $R^1$ denotes a hydrogen atom, a $C_1$-$C_{10}$ alkyl group, or one equivalent of cation; $R^2$ denotes a hydrogen atom or a methyl group; $R^3$ denotes a 2-methylhexyl group or butyl group; n is 0 or 1,and/or enantiomers thereof as active ingredients together with pharmaceutically acceptable carriers or adjuvants.

In the above formula (I)-a $R^1$ signifies a hydrogen atom, a $C_1$-$C_{10}$ alkyl group or one equivalent of cation. Examples of the $C_1$-$C_{10}$ alkyl group expressed by $R^1$ may include straight chain or branched chain types such as methyl, ethyl, n-propyl, iso-propyl, n-butyl, sec-butyl, tert-butyl, n-pentyl, n-hexyl, n-heptyl, n-octyl, n-nonyl, n-decyl, etc. As one equivalent of cation can be given, for example, an alkali metal cation like $Na^+$ or $K^+$, a divalent or trivalent metal cation like 1/2 $Ca^{2+}$, 1/2 $Mg^{2+}$ or 1/3 $Al^{3+}$, an ammonium cation like ammonium ion or tetramethyl ammonium ion, etc. As $R^1$ is especially preferable a hydrogen atom, a methyl group, or a t-butyl group.

In the above formula (I)-a $R^2$ signifies a hydrogen atom or a methyl group. Especially where n=0 a hydrogen atom is preferable, and a methyl group is preferred where n=1.

In the above formula (I)-a $R^3$ stands for a 2-methylhexyl group or butyl group, preferably this is n-butyl, (R)-or (S)- or (RS)-2-methylhexyl group.

As $R^3$ above all, n-butyl and 2-methylhexyl are preferable groups. In the above formula (I)-a n is 0 or 1.

In the case of isocarbacyclins expressed by the above formula (I)-a the carbon atom to which $R^2$, $R^3$ and OH are linked is put in an environment of asymmetric carbon atom. Optical isomers are present therein. If this is explained by giving the case of n=0 as an example, both compounds of the following isocarbacyclins come to exist, i.e. isocarbacyclins having a natural type stereo configuration in the 15-position expressed by the formula [I-1]:

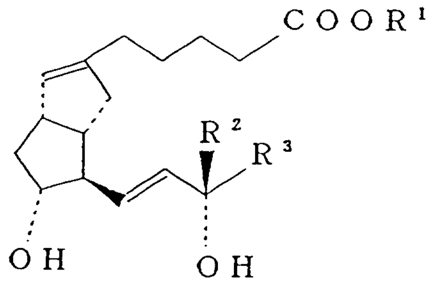

[I-1]

wherein $R^1$, $R^2$ and $R^3$ have the above definitions,

and isocarbacyclins having a non-natural type stereo configuration in the 15-position expressed by the formula [I-2]:

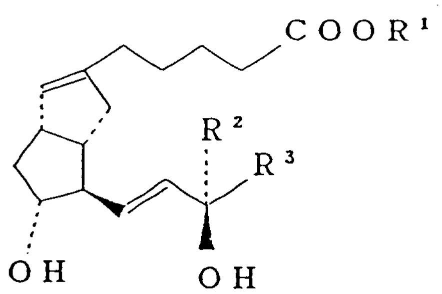

[I-2]

wherein $R^1$, $R^2$ and $R^3$ have the above definitions.

The use application of this patent covers any one of these compounds or a mixture of these compounds in an optional proportion. Especially when $R^2$ is a hydrogen atom, isocarbacyclins expressed by the above formula [I-1] are preferable. The same also applies to the case of n=1 and the use application covers (R)-configuration and (S)-configuration at the 16 position and a mixture thereof in any optional proportion. However, the compound having (S)-configuration is more desirable.

Since stereo configurations in 8-, 9-, 11- and 12 positions of prostacyclins expressed by the above formula (1)-a are the same as those of naturally occurring prostaglandins $I_2$, isomers are especially steric ones. Thus, the present invention includes steric isomers depending on the difference of stereo configurations in respective positions or a mixture thereof having an optional proportion.

As isocarbacyclins of the present invention represented by the above formula (I)-a can be exemplified the following compounds, for instance:

(1) 17,20-dimethyl-9(O)-methano-$\Delta^{6(9\alpha)}$-prostaglandin $I_1$
(2) (17R)-isomer of (1)
(3) (17S)-isomer of (1)
(4) 15-deoxy-16-hydroxy-9(O)-methano-$\Delta^{6(9\alpha)}$-prostaglandin $I_1$
(5) 15-deoxy-16-hydroxy-16-methyl-9(O)-methano-$\Delta^{6(9\alpha)}$-prostaglandin $I_1$
(6) (16S)-isomer of compound (5)
(7) (16R)-isomer of compound (5)
(8) Methyl esters of compounds (1) to (7)
(9) Ethyl esters of compounds (1) to (7)
(10) t-butyl esters of compounds (1) to (7)
(11) Sodium salts of compounds (1) to (7)
(12) Potassium salts of compounds (1) to (7)
(13) Ammonium salts of compounds (1) to (7)
(14) Enantiomers of compounds (1) to (13)
(15) Steric isomers in 8-, 9-, 11-, 12- and 15-positions of compounds (1) to (13)

However, the present invention will not be restricted by these compounds.

Shown below are physical values of several typical compounds inclusive within the formula (I)-a among the above compounds. Further, numbers in parenthesis indicate the above compounds.

(3) (17S)-17,20-dimethyl-9(O)-methano-$\Delta^{6(9\alpha)}$-prostaglandin $I_1$

NMR (CDCl$_3$, $\gamma$ ppm)
0.88 (6H, m), 2.98 (1H, br),
3.75 (1H, q, J=8Hz), 4.13 (1H, q, J=7Hz),
5.29 (1H, bs), 5.47 (1H, dd, J=15H, 8 Hz),
5.50 (1H, dd, J=15H, 8 Hz),
5.95 (3H, br; disappeared in $D_2O$)
IR (liquid film, $cm^{-1}$),
3350, 3400-2400, 1712, 1085, 995, 968.
Mass (m/e)
360 (M-$H_2O$), 342, 316.

(5) 15-deoxy-16-hydroxy-16-methyl-9(O)-methano-$\Delta^{6(9\alpha)\text{-prostaglandin}}$ $I_1$

NMR (CDCl$_3$, $\gamma$ ppm)
0.88 (3H, t, J=6Hz), 1.16 (3H, s),
2.65-3.2 (1H, m), 3.5-3.9 (1H, m),
4.6-5.2 (3H, br; disappeared in $D_2O$),
5.26 (1H, bs), 5.25-5.55 (2H, m).
IR (liquid film, $cm^{-1}$),
3350, 3100-2400, 1705, 965.
Mass (m/e)
346, 329, 328, 302, 246.

(6)-1.....(16S)-isomer of (5)

NMR (CDCl$_3$, $\gamma$ ppm)
0.88 (3H, t, J=6Hz), 1.16 (3H, s),
2.65-3.2 (1H, m), 3.5-3.9 (1H, m),
4.6-5.2 (3H, br; disappeared in $D_2O$),
5.26 (1H, bs), 5.25-5.55 (2H, m).
IR (liquid film, $cm^{-1}$),
3350, 3100-2400, 1705, 965.
Mass (m/e)
346, 329, 328, 302, 246.

(7)-1.....(16R)-isomer of (5)

NMR (CDCl$_3$, $\gamma$ ppm)
0.88 (3H, t, J=6Hz), 1.16 (3H, s),
2.65-3.2 (1H, m), 3.5-3.9 (1H, m),
4.6-5.2 (3H, br; disappeared in $D_2O$)
5.28 (1H, bs), 5.25-5.55 (2H, m).
IR (liquid film, $cm^{-1}$),
3350, 3100-2400, 1705, 965.
Mass (m/e)
346, 329, 328, 302, 246.

(8)-1.....Methyl ester of (3)

NMR (CDCl$_3$, $\gamma$ ppm)
0.88 (6H, m), 2.98 (1H, m), 3.67 (3H, s),
3.75 (1H, m), 4.12 (1H, m), 5.29 (1H, bs),
5.48 (2H, m).
IR (liquid film, $cm^{-1}$),
3380, 1740, 966.

(8)-2.....Methyl ester of (5)

NMR ($CDCl_3$, $\gamma$ ppm)
0.88 (3H, t, J=6Hz), 1.16 (3H, s),
2.7-3.2 (1H, m), 3.5-3.9 (1H, m), 3.66 (3H, s),
5.28 (1H, bs), 5.2-5.6 (2H, m).
IR (liquid film, $cm^{-1}$),
3400, 1740, 968.

(8)-3....Methyl ester of (6)-1

NMR ($CDCl_3$, $\gamma$ ppm)
0.88 (3H, t, J=6Hz), 1.16 (3H, s),
2.7-3.2 (1H, br), 3.5-3.9 (1H, m),
3.67 (3H, s), 5.28 (1H, bs), 5.2-5.6 (2H, m).
IR (liquid film, $cm^{-1}$),
3400, 1740, 968.

(8)-4 .....Methyl ester of (7)-1

NMR ($CDCl_3$, $\gamma$ ppm)
0.88 (3H, t, J=6Hz), 1.16 (3H, s),
2.7-3.2 (1H, br), 3.5-3.9 (1H, m),
3.67 (3H, s), 5.27 (1H, bs), 5.5-5.6 (2H, m).
IR (liquid film, $cm^{-1}$),
3400, 1740, 968.

Prostacyclins $I_1$ (isocarbacyclin) represented by the above formula (I)-a are easily produced in the known way. For instance, the compounds of formula (I)-a can be produced by the process as mentioned in each of European Patent Laid-Open Nos. 216585 and 247740 and US Patent Specification 4788319 corres. to the latter.

Isocarbacyclins of the present invention represented by the above formula (I)-a have a very strong disorder action of organs. For instance, it was clarified by the present invention that these compounds would inhibit the nectrocytosis of hepatocytes induced by carbon tetrachloride or acetoaminophene.

Further, there was confirmed an inhibitory effect of lethal liver nectrocytosis on the autoimmune model mice by the liver antigenic immunity.

Among the isocarbacyclins represented by the above formula (I)-a the compound wherein n=0 is generally strong in the circulatory organ action such as antihypertensive action, action to inhibit the platelet aggregation and the like. There is a possibility that this action may be synergistic to display an action of inhibiting the disorder of organs. While, among the isocarbacyclins expressed by the above formula (I) the compound wherein n=1 holds a strong disorder action of organs, whereas the above circulatory organ action is reduced. Thus, the above compound is characterized by being high in the actional selectivity.

The compounds of the present invention can be administered for the therapy or prevention of disorders of organs such as liver, kidney, pancreas, stomach, heart, lung, etc. and diseases caused by cell disorders.

The instantly claimed compounds can be administered to a patient for the therapy or prevention of his or her acute or chronic hepatic diseases such as toxic hepatopathy disorder, adipohepatic, hepatitis (especially, an alcoholic hepatitis and a viral hepatitis), cirrhosis of the liver, fulminant hepatitis, hepatic coma, hepatomegaly, obstruent jaundice, parastic hepatic diseases, hepatic ulcer, hepatic abscess and so on. The claimed compounds can be also employed as a protecting agent of organs in the hepatic preservation at the time of hepatic transplantation. Further, they can be used as a protecting agent of organs for rejection after transplantation.

Moreover, the instantly claimed compounds can be administered for the therapy or prevention of kidney diseases such as nephritis or diabetic nepropathy, pancreatogeneous diseases such as diabetes mellitus or pancreatitis and diseases of organs like stomach, heart or lung. Where using the compounds of the present invention for the above object, they are administered orally or para-orally like intrarectal, subcutaneous, intramuscular, intravaneous, intra-arterial or transepiderminal administration. Suitably, however, it is good to use these compounds depending on the oral or intravaneous administration.

For the purpose of oral administration they can be made into the form of solid or liquid pharmaceutical preparations. As solid pharmaceutical preparations there is a tablet, a pill, a powder or a granule, for example. In such solid pharmaceutical preparations one or more active substances are mixed with at least one pharmaceutically acceptable carrier such as frequently used sodium bicarbonate, calcium carbonate, potato starch, sucrose, mannitol, carboxymethyl cellulose, etc. An operation of making solid pharmaceutical preparations is conducted in the usual way but there may be also contained additives for making into pharmaceutical preparations other than the foregoing, e.g. a lubricant like as

calcium stearate, magnesium stearate or glycerine. Further, adjuvants can be contained in the solid pharmaceutical preparations as required.

Liquid pharmaceutical preparations for oral administration contain an emulsion, a solution agent, a suspension, a syrup or a xyl agent, for example. These pharmaceutical preparations contain a commonly used pharmaceutically acceptable carrier, e.g. water or a fluid paraffin.

Oily bases such as coconut oil, graduated coconut oil, soybean oil, corn and the like can be used as a carrier.

Pharmaceutical preparations for oral administration can be manufactured as enteric pharmacueticals having an enteric coating, for example, by spraying an organic solvent or a solution in water of enteric substances such as cellulose acetate phthalate, hydroxypropylmethyl cellulose phthalate, polyvinyl alcohol phthalate, a styrene maleic anhydride copolymer, a methacrylic acid or methyl methacrylate copolymer on the above solid pharmaceutical preparations. Enteric solid pharmaceutical preparations such as powder or granule can be wrapped with a capsule.

Examples of the pharmaceutically acceptable carriers include any other adjuvant, aromatic agent, stabilizer or antiseptic which is usually used as required.

Further, liquid pharmaceutical preparations with powder may be administered by enclosing them in a capsule made of any absorbable substance like gelatine.

Solid pharmaceutical preparations for intrarectal administration contain one or more active substances. There are included suppositories produced in the way known per se.

Pharmaceutical preparations for paraoral administration are provided as a sterile, aqueous or non-aqueous spray, a suspension or an emulsion. The non-aqueous solution or suspension contains propyl glycol, polyethylene glycol or a vegetable oil like olive oil and an injectable organic ester like ethyl oleate as the pharmaceutically acceptable carrier. Such pharmaceutical preparations can also contain an adjuvant such as antiseptic, wetting agent, emulsifier, dispersing agent or stabilizer. These spray, dispersing agent and emulsifier can be made sterile, for example, by conducting the following treatment appropriately, i.e. filtration through a bacteria retention filter, formulation of bactericides or irradiation. Further, sterile solid pharmaceutical preparations can be produced and can be also used by dissolving them in a sterile water or a sterile injection solution immediately before their use.

Further, the instanly claimed compounds can be used by forming an inclusion compound together with alpha-beta or gamma-cyclodextrin or metylated cyclodextrin.

As the dosage form of preparations for transepidermal administration can be given an ointment, a gel cream or the like, for example. They can be molded in the usual way.

Isocarbacyclins of the present invention can be administered to a usual adult in an amount of the order 1 g to 10 mg per day where they are used as a therapeutic agent for disorders of organs in vivo. This daily dosage differs according to the symptomatic degree, age, sex, weight of the patient and dosing route, though. Such dosage can be applied by dividing it into one or several portions per day, e.g. 2 to 6 portions.

The present invention will be explained in more detail by way of the following working examples.

## Embodiments

### Example 1

Effect on the necrosis of parenchymal liver cells induced by carbon tetrachloride (invivo)

To each of SD type male rats (six week-age; weight of 170-200g) were administered carbon tetrachloride and a testing medicine in the following procedure. Then the above effect was judged by measuring a glutamic pyruvic acid transaminase (GPT) activity, an index for hepatopathy, in accordance with the ultraviolet portion absorption method (Rate-optimum standard method). Test rats were used in each group of 8 to 36 bodies. The testing medicine was dissolved in a physiological saline solution and administered orally. Every testing medicine was administered five times in total, i.e. 30 minutes before the administration of carbon tetrachloride, 1, 2, 8 and 18 hours after the administration of carbon tetrachloride. Carbon tetrachloride was administered hypodermatically in the form of a 50% olive oil solution at a rate of 0.84 ml/100 g weight (6670 mg/kg weight as carbon tetrachloride). Prior to the administration of carbon tetrachloride, each group of rats was fasted for about 18 hours. Twenty-four hours after the administration of carbon tetrachloride, the rat was etherized and a blood was drawn from its abdominal aorta. Then the blood was allowed to stand at room temperature for one hour and centrifuged at 3000 rpm for 15 minutes. GPT was determined using its supernatant liquid as a serum sample GPT. The result obtained is shown in Table 1. Further, as testing medicines were used (17S)-17,20-dimethyl-9-(O)-methano-$\Delta^{6(9\alpha)}$-prostaglandin $I_1$ [compound (3) ] and 15-deoxy-16-hydroxy-16-methyl-9(O)-methano-$\Delta^{6(9\alpha)}$-prostaglandin $I_1$ [compound (5) ].

Table 1

| Group | GPT (U/ml) | N number |
|---|---|---|
| Intact | 36.6±8.1 | 8 |
| Carbon tetrachloride + vehicle | 3030±1595 | 8 |
| Carbon tetrachloride + compound (3) 1.0 mg/kg | 1682±680 | 8 |
| Carbon tetrachloride + compound (5) 0.3 mg/kg | 1743±592 | 8 |

As is clear from Table 1, the above isocarbacyclins proved to have an inhibitory action of hepatic disorders since they would control a rise of GPT activity involved by carbon tetrachloride.

Example 2

Effect on the necrosis of parenchymal liver cells induced by acetoaminophene (in vivo)

To each ICR type male mouse (five week-age) fasted overnight was administered intracelially 2mM/kg of acetoaminophene suspended in a 5% acacia. A testing medicine was dissolved in a minimal amount of ethanol. The mixture was diluted with a physiological saline solution and was orally administered to the mouse. Further, the testing medicine was administered three times in total, i.e. 30 minutes before the administration of acetoaminophene, one hour and three hours after its administration. Twenty-four hours after administering acetoaminophene a blood was drawn from the mouse and transaminase (GOT and GPT) activities in the serum were determined. The result obtained is shown in Table 2 below.

Further, as testing medicines were used (17S)-17,20-dimethyl-9-(O)-methano- $\Delta^{6(9\alpha)}$-prostaglandin $I_1$ [compound (3)].

Table 2

| Group | GOT* | GPT* | N number |
|---|---|---|---|
| Intact | 48 | 19 | 7 |
| Acetoaminophene + vehicle | 4710 | 5240 | 7 |
| Acetoaminophene + compound (3) 1 mg/kg | 1923 | 2726 | 7 |

Note:
* indicates Kahmen unit.

As is clear from Table 2, the above isocarbacyclin proved to have an inhibitory action of hepatic disorders since it would control a rise of GOT and GPT activities involved by acetoaminophene.

Example 3

Protective effect of parenchymal liver cells from parenchymal liver cell dyscrasic killer T cells

In accordance with the method of M. Ogawa et al. (Liver, vol. 29, No. 12, 1683-1685, 1988) each inbred type 57BL/6 (B6) mouse was immunized with 100,000 g of a supernatant liquid of the liver of the above mouse four times in total a week together with an equivalent amount of Freund's complete adjuvant (FCA). There was prepared an experimental hepatitis model involving any mononuclear cell infiltration and necrosis of parenchymal liver cells centrally in the periportal region. After infecting the mouse with this hepatitis, the sensitized splenoid was obtained from the mouse and was used as an effector cell, while $^{51}Cr$ was taken in the separated parenchymal liver cell from a normal mouse and was employed as a target cell. These target cell and effector cell were mixed at a ratio of 1:100 in the microculture plate and the reaction was conducted for 8 hours under conditions (37°C, 5% $CO_2$). By measuring $^{51}Cr$ liberated from the destructed target cell a study was made about the disorder ability of the effector cell. The result obtained is shown in

Table 3.

Further, as the testing medicine was used (16S)-15-deoxy-16-hydroxy-16-methyl-9-(O)-methano-$\Delta^{6(9\alpha)}$-prostaglandin $I_1$ [compound (6) ].

Table 3

| Kind of the agent | Final concentration | Cell disorder ability (%) |
|---|---|---|
| (-) | | 31.1±4.5* |
| | $10^{-6}$M | 16.8±2.1* |
| | $10^{-7}$ | 18.4±3.2* |
| Compound (6) | | |
| | $10^{-8}$ | 21.1±2.6** |
| | $10^{-9}$ | 23.5±3.7*** |
| | $10^{-11}$ | 29.3±1.9 |
| | $10^{-13}$ | 32.5±3.0 |

* P<0.001,
** P<0.005,
*** P<0.01

As is clear from Table 3, compound (6) proved to have an inhibitory action of hepatic disorders since they would significantly control a cell disorder ability depending on the experimental system of the present case in a dosage dependent manner.

1) Yoshio Mori et al, Clin. Exp. Immunol., 57:85-92, (1984).
2) Teruo Mori et al, Hepatology, 5:770-777 (1985).

Example 4

Protective effect in the autoimmunizable hepatitis model (in vivo)

In accordance with the method of M. Ogawa et al. (Liver, vol. 28, No. 9, 1226-1232, 1987) a fresh liver obtained from each inbred type 57BL/6 (B6) mouse was homogenized with an equivalent amount of a physiological saline solution. The mixture was centrifuged for one hour thereby to afford 100,000 g of a supernatant liquid. This was used as a hepatic antigen. The line of the backbone of each inbred type mouse was immunized with 0.1 mol of the liver antigen together with an equivalent amount of Freund's complete adjuvant in a range of one to four times. On the seventh day from the final immunization 0.5, 1.0 and 2.0 mg/kg of the testing medicine [compound (22)] was administered by means of single phleboclysis. After the lapse of one hour endotoxicin (ET, 25 μg/mouse; colibacillus LPS026: B6, DIFCO Laboratories, Detroit, Michigan, U.S.A.) was dissolved in a physiological saline solution and the mixture was administered to the tail vein. As the control only the physiological saline solution was administered. After its administration, the mouse was observed over a course of 48 hours thereby to calculate the mortality (%). The result obtained is shown in Table 4. Further, the testing medicine [compound (6) ] is the same as the compound used in Example 3.

Table 4

| | | | mortality |
|---|---|---|---|
| Group 1 | The group administered with a physiological saline solution + ET | 3/5 | 60 % |
| Group 2 | Compound (6) (0.5 mg/kg) + ET | 4/5 | 80 % |
| Group 3 | Compound (6) (1.0 mg/kg) + ET | 2/5 | 40 % |
| Group 4 | Compound (6) (2.0 mg/kg) + ET | 0/5 | 0 % |

As is clear from Table 4, compound (6) also proved to have an inhibitory action of hepatic disorders in the vivo testing since it would control the lethal hepatic necrosis involved by this experimental system in a dosage dependent manner.

Example 5

(Pharmaceutical preparation of ampules)

Ampules were produced, each ampule (5 ml) having the following composition:

| | |
|---|---|
| Active ingredient | 200 μg |
| Polyethylene glycol 600 | 200 mg |
| Distilled water | total amount 50 ml |

Namely, polyethylene glycol and an active ingredient were dissolved in water in the presence of nitrogen. The mixture was boiled, cooled in the presence of nitrogen and distilled. A pre-treated water was added to this solution to bring its volume to a given one and the mixturen was filtered under sterile conditions. Its production is carried out in a diffused light.

The filling operation was conducted in an air flow of nitrogen and the disinfection was performed at 121°C for 20 minutes.

As the above active ingredient were used typically (17S)-17,20-dimethyl-9-(O)-methano-$\Delta^{6(9\alpha)}$-prostaglandin $I_1$ [compound (3) ], 15-deoxy-16-hydroxy-16-methyl-9(O)-methano-$\Delta^{6(9\alpha)}$-prostaglandin $I_1$ [compound (5) ] and its (16S)-compound [compound (6)].

Example 6

Tablets were produced, each tablet having the following composition:

| | |
|---|---|
| Active ingredient | 20 μg or 100 μg |
| Lactose | 280 mg |
| Potato starch | 80 mg |
| Polyvinyl pyrrolidone | 11 mg |
| Magnesium stearate | 5 mg |
| | Total: 376 mg |

Namely, the active ingredient, lactose and potato starch were mixed and the mixture was wetted uniformly with a solution of polyvinyl pyrrolidone dissolved in a 20% ethanol uniformly. This was passed through a 20 mm-mesh filter, dried at 45° C and passed again through a 15 mm-mesh filter. The so obtained granule was mingled in magnesium stearate and the mixture was compressed into the form of a tablet.

As the above active ingredient were used typically (17S)-17,20-dimethyl-9-(O)-methano-$\Delta^{6(9\alpha)}$-prostaglandin $I_1$ [compound (3)], 15-deoxy-16-hydroxy-16-methyl-9(O)-methano-$\Delta^{6(9\alpha)}$-prostaglandin $I_1$ [compound (5) ] and its (16S)-compound [compound (6) ].

Example 7

Hard gelatine capsules were produced, each capsule having the following composition:

| | |
|---|---|
| Active ingredient | 20 μg or 100 μg |
| Microcrystalline cellulose | 195 mg |
| Amorphous silicic acid | 5 mg |
| | Total: 200 mg |

Namely, the finely devided active ingredient, microcrystalline cellulose and unpressed amorphous silicic acid were mixed satisfactorily. The resultant mixture was filled in a hard gelatine capsule.

As the above active ingredient were used typically (17S)-17,20-dimethyl-9-(O)-methano-$\Delta^{6(9\alpha)}$-prostaglandin $I_1$ [compound (3)], 15-deoxy-16-hydroxy-16-methyl-9(O)-methano-$\Delta^{6(9\alpha)}$-prostaglandin $I_1$ [compound (5)] and its (16S)-compound [compound (6) ].

Example 8

(17S)-17,20-dimethyl-9-(O)-methano-$\Delta^{6(9\alpha)}$-prostaglandin$I_1$ [compound (3)] was dissolved in a graduated coconut oil. Further, a coating ingredient according to the following recipe was dissolved by warming. Soft capsules were produced in the usual way by using a soft capsule production machine so that one capsule may contain 50 μg of (17S)-17,20-dimethyl-9-(O)-methano-$\Delta^{6(9\alpha)}$-prostaglandin $I_1$.

| Receipe of the coating | |
|---|---|
| Gelatine | 10 wt. parts |
| Glycerine | 5 wt. parts |
| Sorbitan acid | 0.08 wt. part |
| Purified water | 14 wt. parts |

Similarly, soft capsules containing 50 μg of compound (5) were produced by using 15-deoxy-16-hydroxy-16-methyl-9-(O)-methano-$\Delta^{6(9\alpha)}$-prostaglandin $I_1$ [compound (5)] and its (16S)-compound [compound (6) ].

## Claims

### Claims for the following Contracting States : AT, BE, CH, DE, FR, GB, IT, LI, NL, SE

1. A pharmaceutical composition useful for the prevention or therapy of hepatic diseases, kidney diseases, pancreatic diseases and diseases of stomach, heart or lung, which contains isocarbacyclins of the following formula:

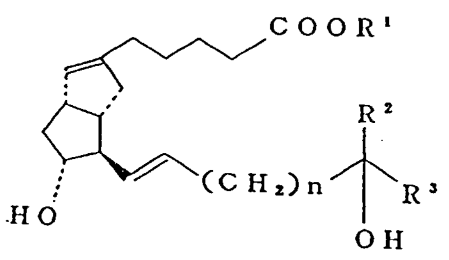

(I-)-a

wherein $R^1$ denotes a hydrogen atom, a $C_1$-$C_{10}$ alkyl group, or one equivalent of cation; $R^2$ denotes a hydrogen atom or a methyl group; $R^3$ denotes a 2-methylhexyl group or butyl group; n is 0 or 1, and/or enantiomers thereof as active ingredients together with pharmaceutically acceptable carriers or adjuvants.

2. A pharmaceutical composition according to claim 1 wherein n is 0, $R^2$ denotes a hydrogen atom, and $R^3$ denotes

a 2-methylhexyl group in the above formula (I-)-a.

3. A pharmaceutical composition according to claim 1 wherein n is 1, $R^2$ denotes a methyl group, and $R^3$ denotes a butyl group in the above formula (I-)-a.

4. A pharmaceutical composition according to claim 3 wherein the isocarbacyclins are (16S)-isomers.

**Claims for the following Contracting State : ES**

1. Process for the production of a pharmaceutical composition useful for the prevention or therapy of hepatic diseases, kidney diseases, pancreatic diseases and diseases of stomach, heart or lung, which comprises mixing at least one isocarbacycline of the following formula:

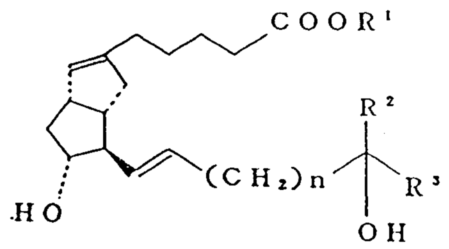

(I-)-a

wherein $R^1$ denotes a hydrogen atom, a $C_1$-$C_{10}$ alkyl group, or one equivalent of cation; $R^2$ denotes a hydrogen atom or a methyl group; $R^3$ denotes a 2-methylhexyl group or butyl group; n is 0 or 1, and/or enantiomers thereof as active ingredients together with a pharmaceutically acceptable carrier or adjuvant.

2. A process for the production of a pharmaceutical composition according to claim 1 wherein n is 0, $R^2$ denotes a hydrogen atom, and $R^3$ denotes a 2-methylhexyl group in the above formula (I-)-a.

3. A process for the production of a pharmaceutical composition according to claim 1 wherein n is 1, $R^2$ denotes a methyl group, and $R^3$ denotes a butyl group in the above formula (I-)-a.

4. A process for the production of a pharmaceutical composition according to claim 3 wherein the isocarbacyclins are (16S)-isomers.

**Patentansprüche**

**Patentansprüche für folgende Vertragsstaaten : AT, BE, CH, DE, FR, GB, IT, LI, NL, SE**

1. Pharmazeutisches Präparat, das zur Prävention oder Therapie von hepatischen Krankheiten, Nierenkrankheiten, Krankheiten des Pancreas und Krankheiten des Magens, des Herzens oder der Lunge geeignet ist, enthaltend Isocarbacycline der folgenden Formel:

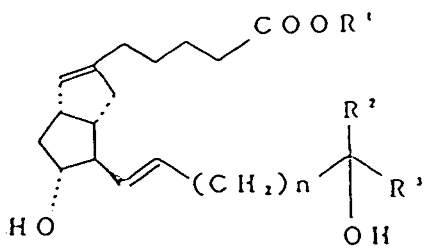

(I-)-a

worin $R^1$ ein Wasserstoffatom, eine $C_1$-$C_{10}$ Alkylgruppe oder ein Äquivalent eines Kations bedeutet; $R^2$ ein Wasserstoffatom oder eine Methylgruppe bedeutet; $R^3$ eine 2-Methylhexylgruppe oder Butylgruppe bedeutet; n gleich 0 oder 1 ist, und/oder Enantiomere davon als Wirkstoffe, zusammen mit pharmazeutisch annehmbaren Trägern oder Adjuvantien.

2. Pharmazeutisches Präparat nach Anspruch 1, dadurch **gekennzeichnet,** daß in der obigen Formel (I-)-a n gleich 0 ist, $R^2$ ein Wasserstoffatom bedeutet und $R^3$ eine 2-Methylhexylgruppe bedeutet.

3. Pharmazeutisches Präparat nach Anspruch 1, dadurch **gekennzeichnet,** daß in der obigen Formel (I-)-a n gleich 1 ist, $R^2$ eine Methylgruppe bedeutet und $R^3$ eine Butylgruppe bedeutet.

4. Pharmazeutisches Präparat nach Anspruch 3, dadurch **gekennzeichnet,** daß die Isocarbacycline (16S)-Isomere sind.

**Patentansprüche für folgenden Vertragsstaat : ES**

1. Verfahren zur Herstellung eines pharmazeutischen Präparats, das zur Prävention oder Therapie von hepatischen Krankheiten, Nierenkrankheiten, Krankheiten des Pancreas und Krankheiten des Magens, des Herzens oder der Lunge geeignet ist, gekennzeichnet durch Mischen mindestens eines Isocarbacyclins der folgenden Formel:

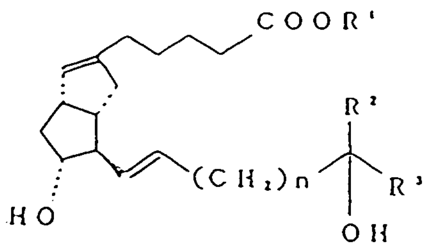

(I-)-a

worin $R^1$ ein Wasserstoffatom, eine $C_1$-$C_{10}$ Alkylgruppe oder ein Äquivalent eines Kations bedeutet; $R^2$ ein Wasserstoffatom oder eine Methylgruppe bedeutet; $R^3$ eine 2-Methylhexylgruppe oder Butylgruppe bedeutet; n gleich 0 oder 1 ist, und/oder Enantiomeren davon als Wirkstoffe, zusammen mit einem pharmazeutisch annehmbaren Träger oder Adjuvans.

2. Verfahren zur Herstellung eines pharmazeutischen Präparats nach Anspruch 1, dadurch **gekennzeichnet,** daß in der obigen Formel (I-)-a n gleich 0 ist, $R^2$ ein Wasserstoffatom bedeutet und $R^3$ eine 2-Methylhexylgruppe bedeutet.

3. Verfahren zur Herstellung eines pharmazeutischen Präparats nach Anspruch 1, dadurch **gekennzeichnet,** daß in der obigen Formel (I-)-a n gleich 1 ist, $R^2$ eine Methylgruppe bedeutet und $R^3$ eine Butylgruppe bedeutet.

4. Verfahren zur Herstellung eines pharmazeutischen Präparats nach Anspruch 3, dadurch **gekennzeichnet,** daß die Isocarbacycline (16S)-Isomere sind.

**Revendications**

**Revendications pour les Etats contractants suivants : AT, BE, CH, DE, FR, GB, IT, LI, NL, SE**

1. Composition pharmaceutique utile pour la prévention ou la thérapie des maladies du foie, des maladies du rein, des maladies du pancréas et des maladies gastriques, cardiaques ou pulmonaires, qui contiennent des isocarbacyclines de formule suivante :

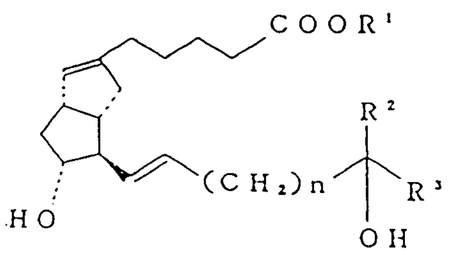

(I-)-a

dans laquelle $R^1$ représente un atome d'hydrogène, un groupe alkyle en $C_1$-$C_{10}$ ou un équivalent de cation ; $R^2$ représente un atome d'hydrogène ou un groupe méthyle ; $R^3$ représente un groupe 2-méthylhexyle ou butyle ; n vaut 0 ou 1 et/ou leurs énantiomères en tant qu'ingrédients actifs conjointement avec des véhicules ou adjuvants pharmaceutiquement acceptables.

**2.** Composition pharmaceutique selon la revendication 1, dans laquelle n vaut 0, $R^2$ représente un atome d'hydrogène, et $R^3$ représente un groupe 2-méthylhexyle dans la formule (I-)-a ci-dessus.

**3.** Composition pharmaceutique selon la revendication 1, dans laquelle n vaut 1, $R^2$ représente un groupe méthyle et $R^3$ représente un groupe butyle dans la formule (I-)-a ci-dessus.

**4.** Composition pharmaceutique selon la revendication 3, dans laquelle les isocarbacyclines sont les isomères (16S).

**Revendications pour l'Etat contractant suivant : ES**

**1.** Procédé pour la préparation d'une composition pharmaceutique utile pour la prévention ou la thérapie des maladies du foie des maladies du rein, des maladies du pancréas et des maladies gastriques, cardiaques ou pulmonaires, qui contiennent des isocarbacyclines de formule suivante :

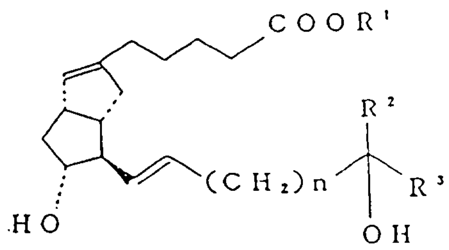

(I-)-a

dans laquelle $R^1$ représente un atome d'hydrogène, un groupe alkyle en $C_1$-$C_{10}$ ou un équivalent de cation ; $R^2$ représente un atome d'hydrogène ou un groupe méthyle ; $R^3$ représente un groupe 2-méthylhexyle ou butyle ; n vaut 0 ou 1 et/ou leurs énantiomères en tant qu'ingrédients actifs conjointement avec des véhicules ou adjuvants pharmaceutiquement acceptables.

**2.** Procédé pour la préparation d'une composition pharmaceutique selon la revendication 1 dans laquelle n vaut 0, $R^2$ représente un atome d'hydrogène, et $R^3$ représente un groupe 2-méthylhexyle dans la formule (I-) -a ci-dessus.

**3.** Procédé pour la préparation d'une composition pharmaceutique selon la revendication 1 dans laquelle n vaut 1, $R^2$ représente un groupe méthyle et $R^3$ représente un groupe butyle dans la formule (I-)-a ci-dessus.

**4.** Procédé pour la préparation d'une composition pharmaceutique selon la revendication 3 dans laquelle les isocarbacyclines sont les isomères (16S).